# EUROPEAN PATENT APPLICATION

(11) **EP 2 149 544 A1**
(43) Date of publication of application: **03.02.2010**
(21) Application number: 08764613.9
(22) Date of filing: 23.05.2008
(51) Int. Cl.: C07C 29/00, C07C 31/38, C07B 61/00

(54) **METHOD FOR PRODUCING FLUOROALKYL ALCOHOL**

(30) Priority: 28.05.2007 JP 2007140727; 28.11.2007 JP 2007307182; 04.03.2008 JP 2008053199
(71) Applicant: Daikin Industries, Ltd., Osaka 530-8323 (JP)
(72) Inventor: SUGIYAMA, Akinari, Settsu-shi Osaka 566-0044 (JP); SUZUKI, Atsushi, Settsu-shi Osaka 566-0044 (JP); TOMITA, Masahiro, Settsu-shi Osaka 566-0044 (JP)
(74) Representative: Luderschmidt, Schüler & Partner
(86) International application number: PCT/JP2008/059567
(87) International publication number: WO 2008/146746

(57) **Abstract**

The present invention provides a method for producing a fluoroalkyl alcohol represented by general formula (6) 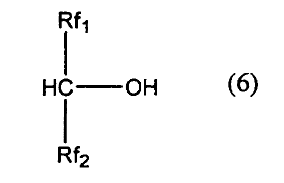 wherein Rf₁ and Rf₂ are the same or different, each represents a CF₃(CF₂)ₙ group (wherein n is an integer of 0 to 10) or a CH₃(CH₂)ₘ group (wherein m is an integer of 0 to 10), and at least one of Rf₁ and Rf₂ represents a CF₃(CF₂)ₙ group,
the method comprising: decarboxylating a compound represented by general formula (1) 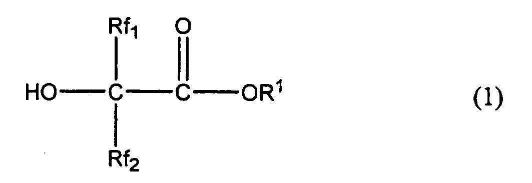 wherein Rf₁ and Rf₂ are the same as above; and R¹ represents an optionally substituted alkyl group, an optionally substituted aryl group, an optionally substituted aralkyl group, an alkali metal, or M_{1/2} (wherein M represents an alkaline earth metal), in the presence of at least one member selected from the group consisting of pyridines, amines, quaternary ammonium salts, and quaternary phosphonium salts.

According to the present invention, fluoroalkyl alcohol such as hexafluoroisopropyl alcohol can be easily produced with high selectivity, using an inexpensive starting material.

## Description

### [Technical Field]

The present invention relates to a method for producing fluoroalkyl alcohol.

### [Background Art]

A known method for preparing hexafluoroisopropyl alcohol is as follows. Octafluoroisobutene is converted to an alcohol adduct, and then subjected to a dehydrofluorination reaction to form (CF₃)₂C=CFOR (heptafluoroisobutenyl alkyl ether), which is then oxidized to form (CF₃)₂C(OH)CO₂R (hydroxycarboxylic ester) or hydroxycarboxylic salt, which is in turn decarboxylated in the presence of a protonic solvent (see PTL 1 below). However, this method problematically requires the use of highly toxic hexafluoroisobutene as a starting material, and results in a low selectivity, i.e., about 70%.
[PTL 1] Japanese Unexamined Patent Publication No. 2002-234860

### [Summary of Invention]

### [Technical Problem]

The present invention was made in view of the problems of the prior art. A primary object of the invention is to provide a method capable of easy and highly selective production of fluoroalkyl alcohol such as hexafluoroisopropyl alcohol, using an inexpensive starting material.

### [Solution to Problem]

To achieve the above object, the present inventors carried out extensive research. As a result, they found the following. When a fluorine-containing hydroxycarboxylic acid obtained from octafluoroisobutene or its similar compound, which is industrial waste and whose efficient use is desired, is used as a starting material and subjected to a decarboxylation reaction in the presence of a specific amine compound, quaternary ammonium salt, or quaternary phosphonium salt, the target fluoroalkyl alcohol can be easily produced with high selectivity. The present invention was accomplished based on this finding.

Specifically, the present invention provides the following production method of fluoroalkyl alcohol.

Item 1. A method for producing a fluoroalkyl alcohol represented by general formula (6) 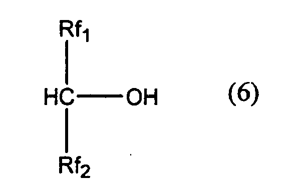 wherein Rf₁ and Rf₂ are the same or different, each represents a CF₃(CF₂)ₙ group (wherein n is an integer of 0 to 10) or a CH₃(CH₂)ₘ group (wherein m is an integer of 0 to 10), and at least one of Rf₁ and Rf₂ represents a CF₃(CF₂)ₙ group,
the method comprising: decarboxylating a compound represented by general formula (1) 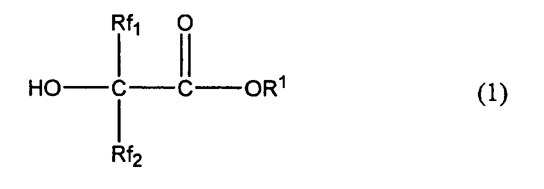 wherein Rf₁ and Rf₂ are the same as above; and R¹ represents an optionally substituted alkyl group, an optionally substituted aryl group, an optionally substituted aralkyl group, an alkali metal, or M_{1/2} (wherein M represents an alkaline earth metal), in the presence of at least one member selected from the group consisting of pyridines represented by general formula (2) 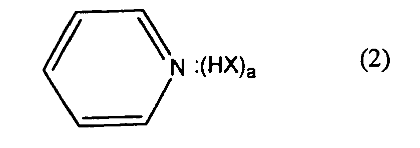 wherein X represents a halogen atom, and a is an integer of 0 to 9,
amines represented by general formula (3) 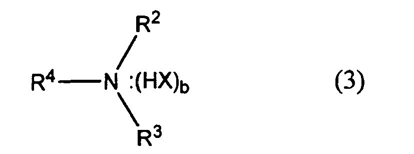 wherein R², R³, and R⁴ are the same or different, and each represents a hydrogen atom, an optionally substituted alkyl group, an optionally substituted aryl group, or an optionally substituted aralkyl group; x represents a halogen atom; and b is an integer of 0 to 9,
quaternary ammonium salts represented by general formula (4) 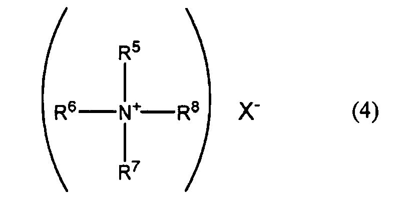 wherein R⁵, R⁶, R⁷, and R⁸ are the same or different, and each represents an alkyl group, a phenyl group, or a benzyl group; and X represents a halogen atom, and
quaternary phosphonium salts represented by general formula (5) 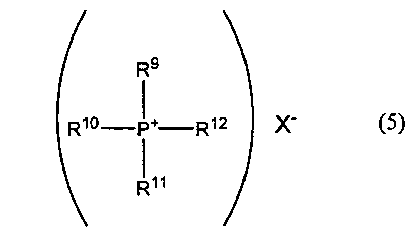 wherein R⁹, R¹⁰, R¹¹, and R¹² are the same or different, and each represents an alkyl group, a phenyl group, or a benzyl group; and X represents a halogen atom.

Item 2. The method for producing a fluoroalkyl alcohol according to Item 1, wherein, in general formula (1), n and m in Rf₁ and Rf₂ are each 0 or 1; and R¹ represents a C₁₋₄ alkyl group, cycloalkyl group, phenyl group, Li, Na, K, or M_{1/2} (wherein M is Mg, Ca, or Ba).

Item 3. The method for producing a fluoroalkyl alcohol according to Item 1 or 2, wherein, in general formula (3), R², R³, and R⁴ are the same or different, and each represents a C₁₋₄ alkyl group.

Item 4. The method for producing a fluoroalkyl alcohol according to any one of Items 1 to 3, wherein,
in general formula (2), X is F, Cl, Br, or I; when R¹ in general formula (1) represents an alkali metal or M_{1/2}, a is 0, and when R¹ represents an optionally substituted alkyl group, an optionally substituted aryl group, or an optionally substituted aralkyl group, a is 1 in the case where X represents Cl, Br or I, or a is an integer of 1 to 9 in the case where X represents F; and
in general formula (3), X is F, Cl, Br, or I; when R¹ in general formula (1) is an alkali metal or M_{1/2}, b is 0, and when R¹ represents an optionally substituted alkyl group, an optionally substituted aryl group, or an optionally substituted aralkyl group, b is 1 in the case where X represents Cl, Br or I, or b is an integer of 1 to 9 in the case where X represents F.

Item 5. The method for producing a fluoroalkyl alcohol according to any one of Items 1 to 4, wherein the decarboxylation reaction is performed in the presence of an amide solvent.

In the present invention, a compound represented by general formula (1) 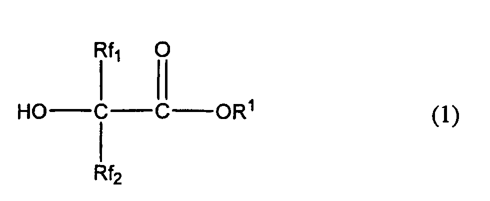 wherein Rf₁ and Rf₂ are the same or different, and each represents a CF₃(CF₂)ₙ group (wherein n is an integer of 0 to 10) or a CH₃(CH₂)ₘ group (wherein m is an integer of 0 to 10), and at least one of Rf₁ and Rf₂ is a CF₃(CF₂)ₙ group; and R¹ represents an optionally substituted alkyl group, an optionally substituted aryl group, an optionally substituted aralkyl group, an alkali metal, or M_{1/2} (wherein M is an alkaline earth metal), is used as a starting material.

In general formula (1) above, Rf₁ and Rf₂ are the same or different, each represents a CF₃(CF₂)ₙ group or a CH₃(CH₂)ₘ group, and at least one of Rf₁ and Rf₂ is a CF₃(CF₂)ₙ group. In particular, it is preferable that both Rf₁ and Rf₂ represent a CF₃(CF₂)ₙ group. In the group, n is an integer of 0 to 10, preferably 0 to 5, and more preferably 0 or 1. Further, m is an integer of 0 to 10, preferably 0 to 5, and more preferably 0 or 1. It is particularly preferable that n be 0, and m be 0 or 1.

R¹ represents an optionally substituted alkyl group, an optionally substituted aryl group, an optionally substituted aralkyl group, an alkali metal, or M_{1/2} (wherein M is an alkaline earth metal). Examples of substituents of the alkyl group, aryl group, and aralkyl group include halogen atoms such as F, Cl, Br, and I, nitro groups, nitrile groups, CH₃(CH₂)_{c}O(CH₂)_{d} groups (wherein c and d are the same or different, and each represents an integer of 0 to 5), CH₃(CH₂)_{c}OCO(CH₂)_{d} groups (wherein e and f are the same or different, and each represents an integer of 0 to 5), CH₃(CH₂)_{g}CO₂(CH₂)ₕ groups (wherein g and h are the same or different, and each represents an integer of 0 to 5), and CH₃(CH₂)ᵢS groups (wherein i is an integer of 0 to 5). Each of the alkyl group, aryl group, and aralkyl group may be substituted with one or more identical or different substituents selected from such examples.

Examples of the alkyl group represented by R¹ include straight or branched lower alkyl groups with about 1 to about 6 carbon atoms, such as methyl, ethyl, n-propyl, isopropyl, n-butyl, isobutyl, sec-butyl, tert-butyl, pentyl, and hexyl; cycloalkyl groups such as cyclohexyl; etc. Examples of the optionally substituted aryl group include phenyl, naphthyl, pyridyl, chlorophenyl, tolyl, nitrophenyl, etc. Examples of the aralkyl group include benzyl, phenethyl, etc.

Examples of the alkali metal include Li, Na, K, Rb, Cs, etc. Li, Na, and K are particularly preferable because they are inexpensive and easily available.

Examples of the alkali earth metal represented by M include Be, Mg, Ca, Sr, Ba, etc. Mg, Ca, and Ba are particularly preferable because they are inexpensive and easily available.

Particularly preferable as R¹ are alkyl groups having about 1 to about 4 carbon atoms, cycloalkyl groups, phenyl groups, alkali metals such as Li, Na, and K, M_{1/2} (wherein M represents an alkaline earth metal such as Mg, Ca, and Ba), etc.

Particularly preferable as the compound represented by general formula (1) are those in which Rf₁ and Rf₂ are both CF₃(CF₂)ₙ groups, and R¹ represents a lower alkyl group having about 1 to about 4 carbon atoms.
The compound represented by general formula (1) is known or similar to known compounds, and can be produced according to the following steps, using as a starting material octafluoroisobutene, which is an industrial waste by-produced during the production of hexafluoropropene.

Specifically, as disclosed in Japanese Unexamined Patent Publication No. 2002-234860, octafluoroisobutene is reacted with alcohol (ROH) to form an alcohol adduct ((CF₃)₂CHCF₂OR) (see F. W. Hoffmann et al.; J. Am. Chem. Soc., 79 (1957) 1741, etc.), and then undergoes a dehydrofluorination reaction (see Japanese Unexamined Patent Publication No. S63-35534). The resulting hexafluoroisobutenyl alkyl ether ((CF₃)₂)C=CFOR) is oxidized to thereby obtain the hydroxycarboxylic ester ((CF₃)₂C(OH)COOR) represented by general formula (1). The oxidation reaction can be carried out by a method using KMnO₄ as an oxidant (see Utebaev U. et al.; Izv. Akad. Nauk SSSR Ser. Khim., 2 (1974) 387), a method using H₂O₂ as an oxidant (see Japanese Unexamined Patent Publication No. S61-286348), etc. The oxidation reaction can also be conducted using an osmium catalyst or ruthenium catalyst (see Japanese Unexamined Patent Publication No. 2002-234860). Other compounds represented by general formula (1) can be obtained in a similar manner.

The production method of the fluoroalkyl alcohol of the present invention is a method comprising the step of decarboxylating the compound represented by general formula (1) above, in the presence of at least one member selected from the group consisting of pyridines represented by general formula (2) 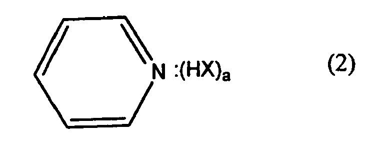 wherein X is a halogen atom, and a is an integer of 0 to 9,
amines represented by general formula (3) 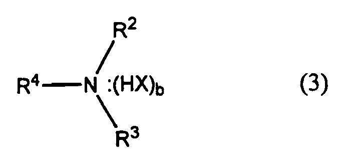 wherein R², R³, and R⁴ are the same or different, and each represents a hydrogen atom, an optionally substituted alkyl group, an optionally substituted aryl group, or an optionally substituted aralkyl group; x represents a halogen atom; and b is an integer of 0 to 9, quaternary ammonium salts represented by general formula (4) 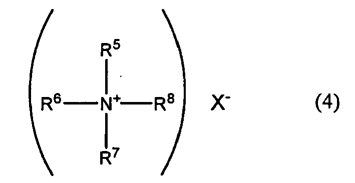 wherein R⁵, R⁶, R⁷, and R⁸ are the same or different, and each represents an alkyl group, a phenyl group, or a benzyl group; and X represents a halogen atom, and quaternary phosphonium salts represented by general formula (5) 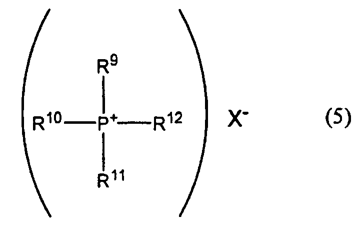 wherein R⁹, R¹⁰, R¹¹, and R¹² are the same or different, and each represents an alkyl group, a phenyl group, or a benzyl group; and X represents a halogen atom.

In the pyridines represented by general formula (2), X represents a halogen atom such as F, Cl, Br, and I. In view of availability, F, Cl, and Br are preferable, and F and Cl are more preferable. The symbol "a" represents an integer of 0 to 9. When R¹ in general formula (1) represents an alkali metal or M_{1/2}, a is preferably 0, and when R¹ represents an optionally substituted alkyl group, an optionally substituted aryl group, or an optionally substituted aralkyl group, a is preferably an integer of 1 to 9. In particular, when R¹ represents an optionally substituted alkyl group, an optionally substituted aryl group, or an optionally substituted aralkyl group, a is preferably 1 in the case where X is Cl, Br or I, or a is preferably an integer of 1 to 9, more preferably 1 to 5, and most preferably 1 to 3 in view of availability in the case where X is F.

In the amines represented by general formula (3), R², R³, and R⁴ are the same or different, and each represents a hydrogen atom, an optionally substituted alkyl group, an optionally substituted aryl group, or an optionally substituted aralkyl group; X represents a halogen atom; and b is an integer of 0 to 9. Among these, preferable as the alkyl group are those having about 1 to about 10 carbon atoms, and particularly preferable are straight or branched lower alkyl groups with about 1 to about 6 carbon atoms, such as methyl, ethyl, n-propyl, isopropyl, n-butyl, isobutyl, sec-butyl, tert-butyl, pentyl, and hexyl; cycloalkyl groups such as cyclohexyl; etc. Preferable as the group represented by R², R³, and R⁴ are a hydrogen atom, methyl, ethyl, n-propyl, isopropyl, n-butyl, isobutyl, sec-butyl, t-butyl, octyl, phenyl, benzyl, etc. X represents a halogen atom such as F, Cl, Br, and I. In view of availability, F, Cl, and Br are preferable, and Cl and Br are more preferable.

In the amines represented by general formula (3), b is an integer of 0 to 9. When R¹ in general formula (1) represents an alkali metal or M_{1/2}, b is preferably 0; and when R¹ represents an optionally substituted alkyl group, an optionally substituted aryl group, or an optionally substituted aralkyl group, b is preferably an integer of 1 to 9. In particular, when R¹ represents an optionally substituted alkyl group, an optionally substituted aryl group, or an optionally substituted aralkyl group, b is preferably 1 in the case where X is Cl, Br, or I, or b is preferably an integer of 1 to 9, more preferably 1 to 5, and most preferably 1 to 3 in view of availability in the case where X is F.

In particular, preferable as the amines represented by general formula (3) are secondary amines or tertiary amines having 0 or 1 hydrogen atom.

In the quaternary ammonium salts represented by general formula (4), R⁵, R⁶, R⁷, and R⁸ are the same or different, and each represents an alkyl group, phenyl group, or benzyl group; and X represents a halogen atom. Among these, preferable as the alkyl group are those having about 1 to about 10 carbon atoms, and particularly preferable are straight or branched lower alkyl groups with about 1 to about 6 carbon atoms, such as methyl, ethyl, n-propyl, isopropyl, n-butyl, isobutyl, sec-butyl, tert-butyl, pentyl, and hexyl; cycloalkyl groups such as cyclohexyl; etc. In particular, preferable examples of R⁵, R⁶, R⁷, and R⁸ include methyl, ethyl, n-propyl, isopropyl, n-butyl, isobutyl, sec-butyl, t-butyl, octyl, phenyl, benzyl, etc. X is a halogen atom such as F, Cl, Br and I. In view of availability, F, Cl, and Br are preferable, and Cl and Br are more preferable.

In the quaternary phosphonium salts represented by general formula (5), R⁹, R¹⁰, R¹¹, and R¹² are the same or different, and each represents an alkyl group, phenyl group, or benzyl group; and X represents a halogen atom. Among these, preferable as the alkyl group are those having about 1 to about 10 carbon atoms, and particularly preferable are straight or branched lower alkyl groups with about 1 to about 6 carbon atoms, such as methyl, ethyl, n-propyl, isopropyl, n-butyl, isobutyl, sec-butyl, tert-butyl, pentyl, and hexyl; cycloalkyl groups such as cyclohexyl; etc. In particular, preferable examples of R⁹, R¹⁰, R¹¹, and R¹² include methyl, ethyl, n-propyl, isopropyl, n-butyl, isobutyl, sec-butyl, tert-butyl, octyl, phenyl, benzyl, etc. X is a halogen atom such as F, Cl, Br, and I. In view of availability, F, Cl, and Br are preferable, and Cl and Br are more preferable.

The amount of at least one member selected from the group consisting of pyridines represented by general formula (2), amines represented by general formula (3), quaternary ammonium salts represented by general formula (4), and quaternary phosphonium salts represented by general formula (5) may be about 0.001 to about 100 mol, preferably about 0.01 to about 50 mol, and more preferably about 0.1 to about 5 mol per mole of the compound of general formula (1), to perform efficient reaction at low cost.

The production method of the present invention can be carried out in the presence or absence of a solvent; however, it is preferably performed in the presence of a solvent in view of the contact efficiency of the reactants. As a solvent, any solvents that are capable of dispersing or dissolving the aforementioned starting materials can be used. Examples of such solvents include hydrocarbon-based solvents such as n-hexane; N-methyl-2-pyrrolidone; dimethylacetamide; nitrobenzene; benzonitrile; dimethylformamide; water; alcohols such as methanol, and ethanol; ethylene glycol; polyethylene glycol; acetonitrile; sulfolane; methylene chloride; chloroform; carbon tetrachloride; dimethylsulfoxide; dimethylacetate; acetic acid; xylene; fluorine-based solvents such as 1,1-dichloro-1-fluoroethane, 3,3-dichloro-1,1,1,2,2,-pentafluoropropane, perfluorohexane, HCF₂CF₂CF₂CF₂Cl, etc. Amines represented by general formula (3) can be also used as solvents. Particularly, when amide solvents such as dimethylacetamide and dimethylformamide are used, the fluoroalkyl alcohol represented by general formula (6) can be obtained with high selectivity.

The amount of solvent, if used, is about 0.001 to about 20 parts by volume, and preferably about 0.01 to about 10 parts by volume, per part by volume of the compound represented by general formula (1). To achieve efficient reaction at low cost, the amount may be about 0.1 to about 5 parts by volume.

There is no limitation on the reaction method. Starting materials may be added at the same time, or may be added separately in an arbitrary order. In particular, to suppress heat generation, it is preferable that any one of the compounds represented by general formulae (2) to (5) be mixed with a solvent, and then the compound represented by general formula (1) be added thereto.

The reaction may be carried out at a temperature of about -20 to about +300°C, but preferably in the range of about 5 to about 200°C.

The reaction can be performed under any pressures such as reduced pressure, atmospheric pressure, and increased pressure, but atmospheric pressure is preferred in view of generation of gas.

The reaction time is generally 0.01 to 50 hours, and preferably about 0.1 to about 24 hours. The components may be added together or separately.

Any material, such as glass and metal, can be used as a material of the reactor, as long as the material has a resistance to the reaction temperature and compound.

According to the method described above, the fluoroalkyl alcohol represented by general formula (6) 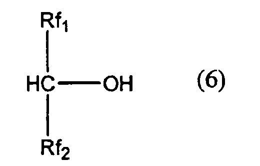 wherein Rf₁ and Rf₂ are the same as above, can be obtained. The resulting fluoroalkyl alcohol can be separated and purified according to known methods. For example, extraction, distillation, recrystallization, chromatography, or the like can be employed.

### [Advantageous Effects of Invention]

According to the present invention, fluoroalkyl alcohol, such as hexafluoroisopropyl alcohol, can be produced with high selectivity and a high yield, using as a starting material a compound represented by general formula (1) obtained from octafluoroisobutene, i.e., industrial waste. Therefore, the method of the present invention is highly effective for inexpensively producing fluoroalkyl alcohol at a high yield.

### [Description of Embodiments]

The present invention is described in further detail with reference to Examples.

### [Example 1]

A 50 ml three-necked flask was charged with 10.0 g (43.1 mmol) of 98% (CF₃)₂C(OH)CO₂CH₃, 6.5 g (47.3 mmol) of triethylamine monohydrochloride, and 10 ml of ethylene glycol.

The flask was placed in a mantle heater, and heated to about 120°C to carry out a reaction. Reflux started as the reaction proceeded, and the temperature was stabilized at about 115°C. After 2 hours of reaction, the reaction mixture was cooled to room temperature, and analyzed. As a result, gas chromatography analysis (GC analysis), gas chromatography/mass analysis (GC/MS analysis), and ¹⁹F-NMR analysis were carried out on water and organic layers.

The results reveal that the conversion ratio of (CF₃)₂C(OH)CO₂CH₃ is 90.9%, and the selectivity of 1,1,1,3,3,3-hexafluoroisopropanol is 77.6%.

### Comparative Example 1

The reaction was conducted in the same manner as in Example 1, but without using triethylamine hydrochloride. As a result, the reaction did not proceed, and hexafluoroisopropanol could not be obtained.

### Example 2

A 50 ml three-necked flask equipped with a reflux condenser was charged with 5.0 g (22.1 mmol) of 99% (CF₃)₂C(OH) CO₂CH₃, 6.7 g (24.3 mmol) of tetra-n-butylammonium chloride, and 5 ml of ethylene glycol. The mixture was heated to 120°C under stirring to carry out a reaction. During the reaction, gas generation was observed. Specifically, methyl chloride and carbon dioxide were generated as gas from the upper part of the condenser. After 2 hours of reaction, the reaction mixture was cooled to room temperature. Gas chromatography analysis(GC analysis), gas chromatography/mass analysis (GC/MS analysis), and ¹⁹F-NMR analysis were carried out on the reaction mixture. The results reveal that the conversion ratio of (CF₃)₂C(OH)CO₂CH₃ is 99%, and the selectivity of 1,1,1,3,3,3-hexafluoroisopropanol is 44%.

### Examples 3 to 6

Decarboxylation reaction and analysis were conducted in the same manner as in Example 2, except that the following material was used in place of tetra-n-butylammonium chloride, in the same mol amount of tetra-n-butylammonium chloride: tetra-n-butylammonium iodide (nBu₄N-I) (Example 3), tetra-n-butylammonium bromide (nBu₄N-Br) (Example 4), triethylbenzylammonium chloride (Et₃NBn-Cl) (Example 5), and triphenylbenzylphosphonium chloride (Ph₃PBn-Cl) (Example 6). The conversion ratio of (CF₃)₂C(OH)CO₂CH₃ and the selectivity of 1,1,1,3,3,3-hexafluoroisopropanol are shown in Table 1.

**Table 1**

| Example | Quaternary ammonium salt or quaternary phosphonium salt | Conversion ratio (%) | Selectivity of hexafluoroisopropanol (%) |
|---|---|---|---|
| 2 | nBu₄N-Cl | 99 | 44 |
| 3 | nBu₄N-I | 99 | 45 |
| 4 | nBu₄N-Br | 99 | 50 |
| 5 | Et₃NBn-Cl | 99 | 53 |
| 6 | Ph₃PBn-Cl | 89 | 51 |

### Examples 7-12

A 50 ml three-necked flask equipped with a reflux condenser was charged with 5.0 g (22.1 mmol) of 99% (CF₃)₂C(OH) CO₂CH₃, 3.3 g (24.3 mmol) of triethylamine hydrochloride, and 5 mL of the solvent shown in Table 2. The mixture was heated to 120°C to conduct a reaction. After the reaction, the analysis was carried out in the same manner as in Example 1, thereby obtaining the conversion ratio of (CF₃)₂C(OH)CO₂CH₃, and the selectivity of 1,1,1,3,3,3-hexafluoroisopropanol. The results are shown in Table 2.

**Table 2**

| Example | Solvent | Conversion ratio (%) | Selectivity of hexafluoro isopropanol (%) |
|---|---|---|---|
| 7 | Acetic acid | 99 | 40 |
| 8 | N-methylpyrrolidone | 99 | 65 |
| 9 | Dimethyl sulfoxide | 99 | 47 |
| 10 | Dimethyl acetate | 99 | 68 |
| 11 | Dimethylformamide | 99 | 79 |
| 12 | Xylene | 99 | 55 |

### Examples 13 and 14

A 50 ml three-necked flask equipped with a reflux condenser was charged with 5.0 g (22.1 mmol) of 99% (CF₃)₂C(OH) CO₂CH₃, 24.3 mmol of amine hydrochloride as shown in Table 3, and 5 mL of N,N-dimethylformamide. The mixture was heated to 120°C to conduct a reaction. After the reaction, the analysis was carried out in the same manner as in Example 1 to obtain the conversion ratio of (CF₃)₂C(OH)CO₂CH₃, and the selectivity of 1,1,1,3,3,3-hexafluoroisopropanol. The results are shown in Table 3.

**Table 3**

| Example | Amine hydrochloride | Conversion ratio (%) | Selectivity of hexafluoro isopropanol (%) |
|---|---|---|---|
| 13 | Diethylamine monohydrochloride | 99 | 82 |
| 14 | Pyridine monohydrochloride | 99 | 83 |

## Claims

1. A method for producing a fluoroalkyl alcohol represented by general formula (6) 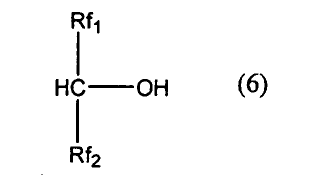 wherein Rf₁ and Rf₂ are the same or different, each represents a CF₃(CF₂)ₙ group (wherein n is an integer of 0 to 10) or a CH₃(CH₂)ₘ group (wherein m is an integer of 0 to 10), and at least one of Rf₁ and Rf₂ represents a CF₃(CF₂)ₙ group,
the method comprising: decarboxylating a compound represented by general formula (1), 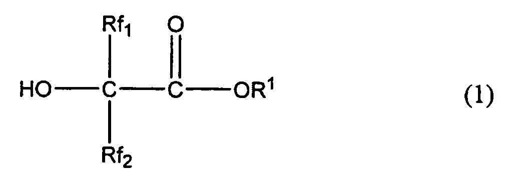 wherein Rf₁ and Rf₂ are the same as above; and R¹ represents an optionally substituted alkyl group, an optionally substituted aryl group, an optionally substituted aralkyl group, an alkali metal, or M_{1/2} (wherein M represents an alkaline earth metal), in the presence of at least one member selected from the group consisting of pyridines represented by general formula (2) 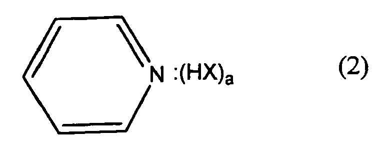 wherein X represents a halogen atom, and a is an integer of 0 to 9,
amines represented by general formula (3) 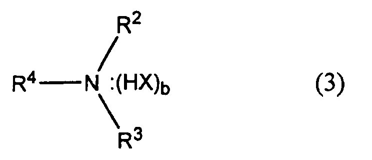 wherein R², R³, and R⁴ are the same or different, and each represents a hydrogen atom, an optionally substituted alkyl group, an optionally substituted aryl group, or an optionally substituted aralkyl group; x represents a halogen atom; and b is an integer of 0 to 9,
quaternary ammonium salts represented by general formula (4) 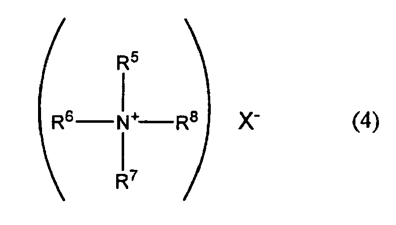 wherein R⁵, R⁶, R⁷, and R⁸ are the same or different, and each represents an alkyl group, a phenyl group, or a benzyl group; and X represents a halogen atom, and
quaternary phosphonium salts represented by general formula (5) 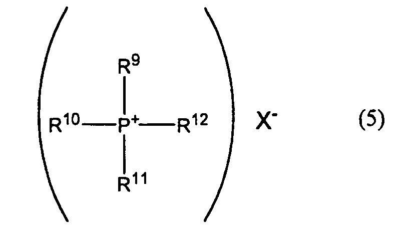 wherein R⁹, R¹⁰, R¹¹, and R¹² are the same or different, and each represents an alkyl group, a phenyl group, or a benzyl group; and
X represents a halogen atom.

2. The method for producing a fluoroalkyl alcohol according to claim 1, wherein, in general formula (1), n and m in Rf₁ and Rf₂ are each 0 or 1; and R¹ represents a C₁₋₄ alkyl group, cycloalkyl group, phenyl group, Li, Na, K, or M_{1/2} (wherein M is Mg, Ca, or Ba).

3. The method for producing a fluoroalkyl alcohol according to claim 1, wherein, in general formula (3), R², R³. and R⁴ are the same or different, and each represents a C₁₋₄ alkyl group.

4. The method for producing a fluoroalkyl alcohol according to claim 1, wherein,
in general formula (2), X is F, Cl, Br, or I; when R¹ in general formula (1) represents an alkali metal or M_{1/2}, a is 0, and when R¹ represents an optionally substituted alkyl group, an optionally substituted aryl group, or an optionally substituted aralkyl group, a is 1 in the case where X represents Cl, Br or I, or a is an integer of 1 to 9 in the case where X represents F; and
in general formula (3), X is F, Cl, Br, or I; when R¹ in general formula (1) is an alkali metal or M_{1/2}, b is 0, and when R¹ represents an optionally substituted alkyl group, an optionally substituted aryl group, or an optionally substituted aralkyl group, b is 1 in the case where X represents Cl, Br or I, or b is an integer of 1 to 9 in the case where X represents F.

5. The method for producing a fluoroalkyl alcohol according to claim 1, wherein the decarboxylation reaction is performed in the presence of an amide solvent.
